# EUROPEAN PATENT APPLICATION

(11) **EP 4 098 741 A1**
(43) Date of publication of application: **07.12.2022**
(21) Application number: 21747437.8
(22) Date of filing: 29.01.2021
(51) Int. Cl.: C12N 7/00, C12N 15/10, A61K 39/12, A61P 31/14, A61K 39/00

(54) **MUTANT STRAIN OF EUROPEAN PORCINE REPRODUCTIVE AND RESPIRATORY SYNDROME VIRUS AND VACCINE COMPOSITION COMPRISING SAME**

(30) Priority: 29.01.2020 KR 20200010636; 28.01.2021 KR 20210012614
(71) Applicant: Biopoa, Inc., Hwaseong-si, Gyeonggi-do 18469 (KR); REPUBLIC OF KOREA (ANIMAL AND PLANT QUARANTINE AGENCY), Gimcheon-si, Gyeongsangbuk-do 39660 (KR)
(72) Inventor: CHO, Sun-Hee, Seongnam-si, Gyeonggi-do 13465 (KR); PARK, Changhoon, Gunpo-si, Gyeonggi-do 15876 (KR); BAEK, Jong Hyuk, Yongin-si, Gyeonggi-do 16963 (KR); CHA, Sang Ho, Cheonan-si, Chungcheongnam-do 31156 (KR); KANG, Seok-Jin, Gimcheon-si, Gyeongsangnam-do 39660 (KR); YOU, Suhwa, Gimcheon-si, Gyeongsangbuk-do 39658 (KR); HYUN, Bang-Hun, Gimcheon-si, Gyeongsangbuk-do 39660 (KR)
(74) Representative: Grünecker Patent- und Rechtsanwälte PartG mbB
(86) International application number: PCT/KR2021/001262
(87) International publication number: WO 2021/154055

(57) **Abstract**

The present invention relates to a mutant strain of European porcine reproductive and respiratory syndrome virus, in which silencing mutation is introduced into a specific site of the genome through codon pair deoptimization, and to a use thereof. The virus mutant strain provided by the present invention is attenuated and decreases in proliferative ability in pigs, compared to the wild-type strain and, as such, can be utilized as a vaccine for prevention of European porcine reproductive and respiratory syndrome.

## Description

### [TECHNICAL FIELD]

The present invention relates to an attenuated mutant of a porcine reproductive and respiratory syndrome virus (PRRSV), and the PRRSV mutant provided by the present invention has lower pathogenicity and higher stability compared to a parent strain.

### [BACKGROUND ART]

Porcine Reproductive and Respiratory Syndrome (hereinafter, referred to as "PRRS") is a viral disease first discovered in the United States in 1987, and was identified in Asia in the early 1990s following Europe. PRRS is a contagious disease that causes the greatest damage to the domestic pig industry along with swine circovirus infectious disease and foot-and-mouth disease, and is spreading worldwide, causing enormous economic loss every year.

PRRS causes reproductive failure such as impotence, miscarriage or premature birth in pregnant sows, and causes respiratory symptoms such as sneezing and fever, and the like in suckling pigs and finishing pigs. In general, severe respiratory symptoms are caused by secondary infection such as bacteria after being infected with a virus, but in the case of chronic infection, a decrease in weight gain and an increase in the mortality rate appear without characteristic clinical symptoms.

The pathogen causing PRRS is a PRRS virus (Porcine Reproductive and Respiratory Syndrome Virus, PRRSV), which belongs to the Arterivirus genus, Arteriviridae family, and Nidovirales order. The PRRS virus is reported that it has positive-sense single stranded RNA (+ssRNA) genome, and its size is about 15.4kb, and it has 9 ORFs. About 80% of the genome is ORFla and ORF1b encoding non-structural protein (NSP), and the remaining 20% consist of ORFs encoding glycosylated structural protein, which are GP2, GP3, GP4 and GP5, and non-glycosylated membrane (M) protein, which is nucleocapsid (N) protein. The minor structural proteins, GP2, GP3 and GP4 form a heterotrimer and act when the virus invades a host cell, and the major structural proteins, GP5 and M form a heterodimer to act to increase infectivity of the virus.

The PRRS virus is highly mutated due to the nature of the RNA virus, so there is a large genetic difference between PRRS viruses. The PRRS virus is largely divided into North American type and European type, and there are Type I (Lelystad virus, LV) representing the North American type and Type II representing the North American strain ATCC VR2332 (See GenBank registration number AY150564 for the genome sequence of VR2332).

It is known that there is a genetic difference of up to 40% between the North American type and European type, and there is no cross-protection against each other. Even among mutants belonging to the same type, cases where cross-protection does not occur have been reported. For this reason, standard mutant-based vaccines have been prepared for each type of virus, but PRRS cannot be effectively prevented due to low cross-protection ability. In order to overcome this, various attempts are being made to produce a vaccine with effective safety, immunogenicity and protective ability.

Due to this limitation, for about 30 years after discovering the porcine reproductive and respiratory syndrome virus (PRRSV), which is the cause of porcine reproductive and respiratory syndrome (PRRS), which causes great damage to the livestock industry, great efforts have been made to develop a prevention method for this virus, but effective prevention and management methods have not yet been developed.

Various vaccines such as inactivated vaccines and attenuated live vaccines have been developed to control the porcine reproductive and respiratory syndrome virus (PRRSV), but only attenuated vaccines with secured infectivity have been found to induce a satisfactory level of protective effect. However, as discussed above, since the porcine reproductive and respiratory syndrome virus (PRRSV) is genetically very diverse, cross-immunity between various viruses is absent, so it is difficult to protect against various porcine reproductive and respiratory syndrome viruses (PRRSV) with one vaccine. In addition, the current attenuated vaccine can only be produced through successive passages of 100~200 or more in cell lines of other animal species, so there is a problem that the development period is long and it is difficult to guarantee efficacy and safety.

### [DISCLOSURE]

### [TECHNICAL PROBLEM]

The present inventors have prepared an attenuated European PRRS virus mutant (PRRSV) using codon pair deoptimization or molecular attenuation technology, thereby completing the present invention.

One embodiment of the present invention provides an attenuated PRRSV mutant.

Another embodiment provides a method for preparation of an attenuated PRRSV mutant.

Other embodiment provides a vaccine composition against PRRSV comprising the attenuated PRRSV mutant.

Other embodiment provides a method for immunization against PRRSV comprising administering the attenuated PRRSV mutant or the vaccine composition into a subject in need of immunization against PRRSV.

Other embodiment provides a use for using in immunization against PRRSV of the attenuated PRRSV mutant or a use for using in preparation of the vaccine composition against PRRSV.

Other embodiment provides a pharmaceutical composition or kit for preventing and/or treating of PRRS comprising the attenuated PRRSV mutant.

Other embodiment provides a method for preparation of a vaccine composition against PRRSV or a pharmaceutical composition for prevention and/or treatment of PRRS using the attenuated PRRSV mutant.

Other embodiment provides a method for prevention and/or treatment of PRRS, comprising administering the attenuated PRRSV mutant or the vaccine composition into a subject in need of prevention and/or treatment of PRRS.

Other embodiment provides a use for using in prevention and/or treatment of PRRS or a use for using in preparation of a pharmaceutical composition for prevention and/or treatment of PRRS, of the attenuated PRRSV mutant or vaccine composition.

The attenuated PRRSV mutant provided by the present invention is safe as a vaccine strain due to reduced proliferation compared to a conventional virus.

### [TECHNICAL SOLUTION]

The present invention relates to a porcine reproductive and respiratory syndrome (PRRS) virus mutant which can be used as a vaccine. The PRRSV virus mutant of the present invention is attenuated than a parent strain, so it has low pathogenicity and high stability. Accordingly, it can be used as an effective vaccine for prevention and treatment of PRRS disease.

Hereinafter, the present invention will be described in more detail.

"Attenuated virus" used in the present description refers to a non-toxic virus capable of inducing an immune response in a target mammal without causing clinical signs of PRRS disease, and also refers to one which is infected by an attenuated virus and reduces the frequency of clinical signs in an animal which is not administered by the attenuated virus, or reduces the severity of symptoms compared to a "control" animal infected by a non-attenuated PRRS virus. In this circumstance, the term "reduce/reduced" means a reduction of at least 10%, preferably 25%, more preferably 50% and most preferably 100% or more, compared to the control group defined above.

"Vaccine composition" used in the present description may be a PRRS chimeric virus or any immunogenic fragment or fraction thereof, preferably, an attenuated PRRS chimeric virus, for example, the PRRS chimeric virus of the present invention. This induces an "immunological response" of a host as an immune response mediated by a cell and/or an antibody against PRRSV. It is preferable that the vaccine composition can give preventive immunity for PRRSV infection and clinical symptoms related thereto.

"Immune response" used in the present description means any cell- and/or antibody-mediated immune response against a chimeric virus or vaccine administered into an animal in which the PRRSV chimeric virus of the present invention or a vaccine composition comprising the same is administered. In common, "immune response" comprises one or more of the following effects, but not limited thereto: production or activation of an antigen comprised in the corresponding composition or vaccine or an antibody induced specifically for antigens, a B cell, a helper T cell, an inhibitory factor T cell and/or a cytotoxic T cell and/or a γδ T cell. Preferably, the host exhibits a therapeutic or prophylactic immunological response such that resistance to new infection is improved and/or the clinical severity of disease is reduced compared to a control group not administered with an immunogenic composition or vaccine. Such prevention may be verified by lack of symptoms associated with the aforementioned host infection and including this, reduction of frequency or severity at most.

"Pigs", "pig" and "piglet" used in the present description may be interchangeably used.

"Inoculate a vaccine" means administering the PRRSV chimeric virus or vaccine comprising the same described in the present description before exposed to PRRS disease.

"Prevent" or "prevention" means that the frequency of clinical incidence of PRRS, and severity or frequency of symptoms of PRRS, as the result of being administered with the PRRSV virus or vaccine composition comprising the same of the present invention. The reduction of the severity or frequency is the result of comparing with an animal or animal group to which the PRRSV chimeric virus or vaccine composition comprising the same of the present invention is not administered. The animal may be preferably a pig.

The nucleotide sequence of the present description is described based on DNA nucleotide for convenience, and when the type of polynucleotide is RNA, it means a sequence in which all or a part of thymine (T) is substituted with uracil (U) in the nucleotide sequence.

In the present description "consisting of a sequence" of a specific nucleotide sequence and/or amino acid includes comprising the sequence, essentially comprising the sequence, and/or consisting of the sequence all, and it may be appropriately substituted and used, if necessary.

The present invention provides a mutant of an attenuated porcine reproductive and respiratory syndrome (PRRS) virus, and the mutant may comprise a silent mutation in the ORF7 region of the porcine reproductive and respiratory syndrome (PRRS) virus.

The ORF7 region is OFR located closest to the 3'-end of the porcine reproductive and respiratory syndrome virus genome, and is known to express a structural protein of PRRSV. When the silent mutation occurs outside of the ORF7 region, production of the virus may not be achieved. In one specific embodiment, as the result of introducing a silent mutation into a gene encoding NSP1 protein in the ORF1a region, production of the virus was not achieved.

The ORF7 sequence of the PRRSV mutant provided by the present invention may have lower codon pair bias (CPB) compared to the OFR7 region of the wild-type PRRSV. The ORF7 region of the wild-type PRRSV may consist of the nucleic acid sequence of SEQ ID NO: 2.

The codon pair bias may be calculated using a program which may be used for a CPB calculation purpose known in the art, for example, SAVE (Synthetic Attenuated Virus Engineering) program, but not limited thereto.

The codon pair bias is changed when some nucleotide sequences of a gene are substituted with other nucleotides, and is particularly closely related to proliferation of the virus. Specifically, when the CPB numerical value is reduced, the proliferation of the virus is reduced and attenuated.

The CPB numerical value of the ORF7 region of the attenuated PRRSV mutant provided by the present invention may show a value 0.05 to 0.5, 0.05 to 0.3, or 0.05 to 0.2 lower than the CPB numerical value of the ORF7 region of the wild-type PRRSV, but not limited thereto.

The CpB and/or UpA numerical values of the ORF7 region of the attenuated PRRSV mutant provided by the present invention are higher than a parent strain. The change of the CpG, UpA ratio is the result necessarily occurring in the deoptimization process, and when the CpG, UpA numerical values of a eukaryote gene are increased, cell stress may be caused and proliferation of the virus may be reduced, thereby attenuating the virus. In one embodiment, the CpB and/or UpA numerical values of the attenuated PRRSV mutant provided by the present invention may have a numerical value of 1 to 5 times, 1 to 3 times, 1.1 to 5 times, 1.1 to 3 times, 1.3 to 5 times, or 1.3 to 3 times, compared to a parent strain, but not limited thereto.

In one embodiment, the CpG numerical value of the ORF7 region of the attenuated PRRSV mutant of the present invention may be 0.7 to 1.5, 0.7 to 1.3, or 0.7 to 1.1, and the UpA numerical value of the ORF7 region of the mutant may be 0.3 to 1.0, 0.3 to 0.9 or 0.3 to 0.8, but not limited thereto.

In one embodiment, the ORF7 sequence of the PRRSV mutant provided by the present invention may comprise a silent mutation at the position after the 177th nucleotide from the 5' end of the ORF7 region consisting of the nucleic acid sequence of SEQ ID NO: 2 or SEQ ID NO: 5. The silent mutation may be freely introduced within a range of a purpose to lower CPB of the ORF7 region, and for example, 7 to 30, or 7 to 22 nucleotides may be substituted. When silent mutations less than 7 are introduced, a virus may not be attenuated enough, and when the number of silent mutations is too large, production of the virus may not be achieved. When the number of silent mutations is larger than the upper limit, it may be excessively attenuated, and therefore, virus survival may be impossible.

In one embodiment, the ORF7 region of the PRRSV mutant provided by the present invention may consist of the nucleic acid sequence of SEQ ID NO: 3, SEQ ID NO: 5, SEQ ID NO: 9 or SEQ ID NO: 10.

In one embodiment, the ORF7 region of the PRRSV mutant provided by the present invention may comprise at least the following mutations in the ORF7 region consisting of the nucleic acid sequence of SEQ ID NO: 2: it may comprise mutation consisting of 7 or more, 14 or more, 16 or more, 18 or more, or 22 or more mutations, selected from the group consisting of mutations in which C at the 123th position is substituted with T, A at the 129th position is substituted with C, T at the 177th position is substituted with C, T at the 180th position is substituted with C, T at the 186th position is substituted with C, C at the 189th position is substituted with T, C at the 204th position is substituted with T, T at the 213th position is substituted with G, C at the 222th position is substituted with G, A at the 264th position is substituted with C, A at the 267th position is substituted with T, T at the 288th position is substituted with C, G at the 291th position is substituted with T, C at the 297th position is substituted with T, T at the 309th position is substituted with C, C at the 315th position is substituted with T, T at the 327th position is substituted with C, T at the 330th position is substituted with G, A at the 336th position is substituted with G, G at the 339th position is substituted with A, T at the 348th position is substituted with A, and G at the 354th position is substituted with T.

More specifically, the ORF7 of the attenuated porcine reproductive and respiratory syndrome virus (PRRSV) mutant provided by the present invention may consist of a nucleic acid sequence in which the nucleic acid sequence of SEQ ID NO: 2 is mutated by a silent mutation in the region after the 177th nucleotide, and have lower codon pair bias (CPB) compared to the porcine reproductive and respiratory syndrome (PRRS) virus.

More specifically, the ORF7 of the attenuated PRRSV mutant provided by the present invention may consist of a nucleic acid sequence in which the nucleic acid sequence of SEQ ID NO: 2 is mutated by one or more mutations selected from the following:
substitution of T at the 177th position with C, substitution of T at the 180th position with C, substitution of T at the 186th position with C, substitution of C at the 189th position with T, substitution of C at the 204th position with T, substitution of T at the 213th position with G, substitution of C at the 222th position with G, substitution of A at the 264th position with C, substitution of A at the 267th position with T, substitution of T at the 288th position with C, substitution of G at the 291th position with T, substitution of C at the 297th position with T, substitution of T at the 309th position with C, substitution of C at the 315th position with T, substitution of T at the 327th position with C, substitution of T at the 330th position with G, substitution of A at the 336th position with G, substitution of G at the 339th position with A, substitution of T at the 348th position with A, and substitution of G at the 354th position with T.

More specifically, the ORF7 of the attenuated PRRSV mutant provided by the present invention ay consist of a nucleic acid sequence in which the nucleic acid sequence of SEQ ID NO: 2 is mutated by the following mutation:
(1) a mutation including one or more (for example, 1, 2, 3, 4, 5, 6 or 7) substitutions selected from the group consisting of substitution of T at the 177th position with C, substitution of T at the 180th position with C, substitution of C at the 204th position with T, substitution of T at the 213th position with G, substitution of C at the 222th position with G, substitution of A at the 264th position with C, and substitution of A at the 267th position with T; or
(2) a combination of (i) the mutation of (1) above, and (ii) one or more mutations selected from the group consisting of substitution of T at the 186th position with C, substitution of C at the 189th position with T, substitution of T at the 288th position with C, substitution of G at the 291th position with T, substitution of C at the 297th position with T, substitution of T at the 309th position with C, substitution of C at the 315th position with T, substitution of T at the 327th position with C, substitution of T at the 330th position with G, substitution of A at the 336th position with G, substitution of G at the 339th position with A, substitution of T at the 348th position with A, and substitution of G at the 354th position with T.

In other embodiment, the ORF7 of the attenuated PRRSV mutant provided by the present invention may consist of a nucleic acid sequence in which the nucleic acid sequence of SEQ ID NO: 2 is further mutated by substitution of the 123th C with T, substitution of the 129th A with C, or a combination thereof.

The parent strain of the PRRSV mutant may be a European porcine reproductive and respiratory syndrome virus, and in one embodiment, it may be E38 strain. The PRRSV mutant provided by the present invention may be classified by the European porcine reproductive and respiratory syndrome virus (Type 1).

The attenuated PRRSV mutant provided by the present invention may have a TCID₅₀ value of 0.99 times or less, 0.95 times or less, 0.7 times or less, 0.6 times or less, or 0.5 times or less, when infected by an alveolar macrophage compared to the parent strain, but not limited thereto. In one example, as the result of measuring the TCID₅₀ value by infecting the E38 virus and the mutant in which a mutation was introduced into the virus into an alveolar macrophage, respectively, a low TCID₅₀ value was exhibited compared to the parent strain in all the mutants, and thereby, low proliferation was confirmed. In one example, the virus mutant having the ORF7 sequence consisting of the nucleic acid sequence of SEQ ID NO: 10, showed a TCID₅₀/mL value at a level of about 65% compared to the parent strain when inoculated into an alveolar macrophage (PAM), and at Day 1 after inoculation, and showed a TCID₅₀/mL value at a level of about 35% at Day 4 after inoculation, and therefore, significantly low toxicity was confirmed compared to the parent strain.

The attenuated PRRSV mutant provided by the present invention may show a blood virus titer of 95% or less, 90% or less, 80% or less, 70% or less, 65% or less, 60% or less, 55% or less, 50% or less, 45% or less or 35% or less, when infected into a pig, compared to the titer (Log (genomic copies/ml)) of the wild-type strain when infected with the virus. In one example, as the result of infecting the E38 virus and the mutant in which a mutation was introduced into the virus into a pig, respectively, all the mutants showed a lower virus titer compared to the parent strain, and thereby, it was confirmed that a safe strain with a low maximum titer capable of proliferating in a pig's body and rapidly disappearing was produced.

The present invention also provides a preparation method of an attenuated porcine reproductive and respiratory syndrome virus (PRRSV).

The preparation method of an attenuated PRRSV provided by the present invention comprises introducing a silent mutation at a position after the 177^{th} nucleotide from the 5'end of ORF7 region in genome of a wild-type PRRSV; and producing a virus (mutant) in which the mutation is introduced.

The 5' end of ORF7 region, means a region corresponding to the 5' end of a polynucleotide consisting of the nucleic acid sequence of SEQ ID NO: 2 in the PRRSV genome nucleic acid sequence. When the silent mutation is introduced at a position other than ORF7, there may be a problem in that production of the virus is not performed.

The silent mutation introduced into the mutant may lower the CPB value of ORF7 region of the mutant compared to ORF7 region of the wild-type virus. Description for the CPB is same as aforementioned.

The position and number and type of the silent mutation are same as those of the silent mutation introduced into the virus mutant.

The wild-type PRRSV may be a European (Type 1) porcine reproductive and respiratory syndrome virus, and in one embodiment, it may be E38 strain.

In one embodiment, the preparation method of an attenuated porcine reproductive and respiratory syndrome virus (PRRSV) provided by the present invention may comprise

Introducing a silent mutation at a position after a base corresponding to the 177^{th} from the 5' end based on the nucleic acid sequence of SEQ ID NO: 2 of ORF7 region in genome of a wild-type porcine reproductive and respiratory syndrome virus; and
producing a virus in which the mutation is introduced.

More specifically, for the preparation method of an attenuated porcine reproductive and respiratory syndrome virus (PRRSV) provided by the present invention, the silent mutation may comprise 7 or more mutations selected from the group consisting of mutations of substitution of C at the 123th position with T, substitution of A at the 129th position with C, substitution of T at the 177th position with C, substitution of T at the 180th position with C, substitution of T at the 186th position with C, substitution of C at the 189th position with T, substitution of C at the 204th position with T, substitution of T at the 213th position with G, substitution of C at the 222th position with G, substitution of A at the 264th position with C, substitution of A at the 267th position with T, substitution of T at the 288th position with C, substitution of G at the 291th position with T, substitution of C at the 297th position with T, substitution of T at the 309th position with C, substitution of C at the 315th position with T, substitution of T at the 327th position with C, substitution of T at the 330th position with G, substitution of A at the 336th position with G, substitution of G at the 339th position with A, substitution of T at the 348th position with A, and substitution of G at the 354th position with T, based on the nucleic acid sequence of SEQ ID NO: 2.

More specifically, for the preparation method of an attenuated porcine reproductive and respiratory syndrome virus (PRRSV) provided by the present invention, the silent mutation may be
(1) a mutation including substitution of T at the 177th position with C, substitution of T at the 180th position with C, substitution of C at the 204th position with T, substitution of T at the 213th position with G, substitution of C at the 222th position with G, substitution of A at the 264th position with C, and substitution of A at the 267th position with T; or
(2) a combination of (i) the mutation of (1) above, and (ii) one or more mutations selected from the group consisting of substitution of T at the 186th position with C, substitution of C at the 189th position with T, substitution of T at the 288th position with C, substitution of G at the 291th position with T, substitution of C at the 297th position with T, substitution of T at the 309th position with C, substitution of C at the 315th position with T, substitution of T at the 327th position with C, substitution of T at the 330th position with G, substitution of A at the 336th position with G, substitution of G at the 339th position with A, substitution of T at the 348th position with A, and substitution of G at the 354th position with T.
based on the nucleic acid sequence of SEQ ID NO: 2.

In other embodiment, for the preparation method of an attenuated porcine reproductive and respiratory syndrome virus (PRRSV) provided by the present invention, the silent mutation may further comprise substitution of C at the 123th position with T, substitution of A at the 129th position with C, or a combination thereof, based on the nucleic acid sequence of SEQ ID NO: 2.

In the preparation method of an attenuated porcine reproductive and respiratory syndrome virus (PRRSV), the porcine reproductive and respiratory syndrome virus may be a European porcine reproductive and respiratory syndrome virus, but not limited thereto.

In the introducing a silent mutation, those skilled in the art may select and use an appropriate method without limitation within a scope of the purpose for introducing a point mutation, and for example, synthesis of a nucleotide sequence, use of a primer pair in which a mutation is introduced, and/or a method of using a commercialized kit, but not limited thereto.

In one example, for an infectious clone of the PRRSV virus mutant in which a mutation is introduced, an infectious clone of a virus mutant may be prepared by performing synthesizing DNA corresponding to each fragment, respectively, by dividing genome of a virus into a plurality of, for example, 2 to 10, 2 to 8.3 to 10, 3 to 8, 5 to 10, or 5 to 8 fragments; and linking each of the fragments. In one example, for an infectious clone of the PRRSV virus mutant in which a mutation is introduced, an infectious clone of a virus mutant was prepared by synthesizing DNA corresponding to each fragment, respectively, by dividing genome of the virus into 6 fragments, and then linking each fragment.

In one embodiment, the linking each fragment may comprise treating the prepared each fragment with restriction enzyme and linking with ligase.

The present invention also provides a pharmaceutical composition for prevention or treatment of porcine reproductive and respiratory syndrome, comprising an attenuated PRRSV mutant and/or subcultured offspring of the mutant.

The content of the PRRSV mutant is same as aforementioned.

The porcine reproductive and respiratory syndrome may occur by a European (Type 1) porcine reproductive and respiratory syndrome virus.

The subcultured offspring may comprise an offspring virus of the virus mutant subcultured by 1 to 80 passages, 1 to 70 passages, 1 to 60 passages, 1 to 50 passages, 1 to 40 passages, 1 to 30 passages, 1 to 20 passages, or 1 to 10 passages.

The composition for prevention or treatment of porcine reproductive and respiratory syndrome of the present invention may comprise an additional component known in the art, and it may further comprise an appropriate carrier, an excipient and a diluent commonly used for preparation of a pharmaceutical composition.

The carrier, excipient and diluent which may be comprised in the pharmaceutical composition of the present invention includes lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate and mineral oil and the like. When the composition is formulated, it is prepared using a diluent or excipient such as a filler, a thickener, a binder, a wetting agent, a disintegrating agent, a surfactant, and the like. A solid preparation for oral administration includes tablet, pill, powder, granule, capsule and the like, and this solid preparation is prepared by mixing at least one or more excipients, for example, starch, calcium carbonate, sucrose, lactose, gelatin and the like to the composition. In addition, lubricants such as magnesium stearate and talc in addition to the simple excipient are used. A liquid preparation for oral administration includes suspending agent, oral liquid, emulsion, syrup, and the like, and various excipients, for example, wetting agent, sweetener, flavoring agent, preservative, and the like may be comprised in addition to commonly used simple diluents, water and liquid paraffin. A preparation for parenteral administration includes sterilized aqueous solution, nonaqueous liquid, suspending agent, emulsion, freeze-drying agent and suppository. As the nonaqueous liquid and suspending agent, propylene glycol, polyethylene glycol, vegetable oil such as olive oil, injectable ester such as ethyl oleate, and the like may be used. As a base compound of the suppository, Witepsol, macrogol, Tween61, cacao butter, laurin fat, glycerogelatin and the like may be used.

The preferable dosage of the composition of the present invention varies depending on the condition and body weight of a subject, degree of disease, drug form, administration route and period, but may be appropriately selected by those skilled in the art. For example, for a preferable effect, the composition of the present invention may be administered in an amount of 0.0001 to 1,000 mg/kg (body weight) per day. Administration of the composition may be administration once a day or divided into several administrations.

In one embodiment, the pharmaceutical composition may be a vaccine composition. The vaccine may be a live vaccine and/or a dead vaccine, and specifically, the attenuated PRRS chimeric virus described herein may be a modified live vaccine containing one or more of the aforementioned virus strains in a live condition in a pharmaceutically acceptable carrier. In addition, or alternatively, an inactivated virus may be used for preparing a dead vaccine.

The vaccine may comprise the PRRSV mutant at an appropriate concentration in consideration of the body weight, age, diet stage and/or immunity of a subject for administration within a range of a PRRS preventive purpose. For example, the dosage of the virus mutant in the vaccine composition may be in a range of TCID₅₀ 2 to 6, or TCID₅₀ 3 to 4, but it may differ depending on the kind of the subject, and is not limited thereto.

The vaccine composition of the present invention may be administered to a pig, and the pig may be a pig in one or more growth stages selected from the group consisting of weaning piglet stage, growing stage and finishing stage. The pig of the weaning piglet stage means a pig from 7 days or more, 14 days or more, or 21 days or more, after birth, to a weight of 30 kg, and the growing stage may mean a period when the body weight of the pig is 30 to 50kg, and the finishing stage may mean a period after the growing stage. The pig may be a hog raising pig and/or a wild boar, but does not discriminate the breed, but for example, it may be one kind or more, 2 kinds or more, 3 kinds or more, 4 kinds or more, or 5 kinds or more selected from the group consisting of Landrace species, Yorkshire species, Duroc species, Berkshire species and Korean native pigs, and includes all pigs born by crossbreeding between the species.

The vaccine may further comprise one or more selected from the group consisting of a carrier, a diluent, an excipient and an adjuvant. The pharmaceutically acceptable carrier is not particularly limited in its type, but may include any all solvents, dispersion media, coatings, stabilizers, preservatives, antibacterial and antifungal agents, isotonic agents, absorption delaying agents, and the like.

The attenuated PRRSV mutant of the present invention, or a vaccine composition comprising the same may be administered by oral, parenteral, subcutaneous, intramuscular, intradermal, sublingual, dermal, intrarectal, transmucosal, surface through inhalation, or buccal administration, or a combination thereof. In addition, the attenuated PRRSV mutant may be administered in an implant form capable of allowing sustained release of the attenuated virus.

The attenuated PRRSV mutant of the present invention or a vaccine composition comprising the same may be administered by injection, inhalation or transplantation, but not limited thereto. Depending on the desired period and effectiveness of vaccination or treatment, the attenuated PRRSV mutant or vaccine composition comprising the same may be administered once or several times, and also intermittently, for example, in the same amount or in different doses daily for several days, several weeks or several months. An injection may be injected in a desired amount or be injected by subcutaneous or nasal spray, or alternatively, may be continuously injected.

In one embodiment, the pharmaceutical composition may be provided in a form of a kit for preventing or treating porcine reproductive and respiratory syndrome. The kit may comprise a container, preferably, a vaccine composition containing the attenuated PRRS chimeric virus of the present invention, a pharmaceutically acceptable carrier, an adjuvant and instructions for use for administering the immunogenic composition into an animal in need thereof to alleviate clinical signs or effects of PRRS infection, preferably, the frequency or severity of PRRS. The kit may also comprise injection means and/or other forms of administration means. In addition, the kit may comprise a solvent. The attenuated vaccine may be lyophilized, and it may be a solution for injection and/or inhalation by being restored with a solvent. The solvent may be water, physiological saline solution, buffer or an adjuvant solvent. The kit may comprise a separate container containing the attenuated virus, solvent and/or pharmaceutically acceptable carrier. The instructions for use may be labels and/or printed materials affixed to one or more containers.

### [ADVANTAGEOUS EFFECTS]

The mutant of a porcine reproductive and respiratory syndrome virus provided by the present invention is attenuated than a parent strain, so it has low pathogenicity and high stability, and improves porcine immunity, and therefore, it can be used as an effective vaccine for prevention and/or treatment of PRRS disease.

### [BRIEF DESCRIPTION OF THE DRAWINGS]

FIG. 1 is the result of aligning and comparing the nucleic acid sequence of the ORF7 region of E38 parent strain and each mutant. The color of the circle in front of the SEQ ID NO legend indicates success or failure of construction of a virus comprising ORF7 of each nucleic acid sequence (blue: construction success, red: construction failure).
FIG. 2 is a graph showing the result of confirming the proliferation of the virus over time after infecting each constructed virus into a porcine lung macrophage PAM).
FIG. 3 is a graph showing the virus titer in blood over time after inoculating each constructed virus into a pig. ^{∗} indicates a statistically significant result.

### [MODE FOR INVENTION]

Hereinafter, the present invention will be described in more detail by examples. However, the following examples are intended only to illustrate the present invention, but the scope of the present invention is not limited by the following examples.

In the following examples, the genome of PRRSV, RNA is easily destroyed, unless otherwise mentioned, all PRRSV genome processing was conducted by performing an operation by synthesizing DNA, and then RNA was prepared therefrom and used for transfection of cells.

### Preparative example 1. Parent strain information

As a parent strain for preparing a mutant, a European PRRSV isolated within the country, E38 strain was provided from Quarantine Agency (deposited to Korea Veterinary Culture Collection (http://kvcc.kahis.go.kr/pms/web/gene/pmsGeneMain.do?iNo=3*&*iType=01*&*iSubType=01*&*sea rchKeyword1=*&*searchKeyword2=vr1800004*&*searchKeyword3=*&*searchCondition=0*&*search Keyword=vr1800004*&*searchSale=0); Genetic resource number KVCC-VR1800004), and the entire genome sequence of the E38 strain was shown in SEQ ID NO: 1.

The result of comparing the entire genome sequence homology of the domestic isolated species E38 with American type PRRSV (VR-2332) and European PRRSV (Amervac, Lelystad and Lena strains) was shown in Table 1 below.

**[Table 1]**

| PRRSV type | North American type (Type II) | European type (Type I) | | |
|---|---|---|---|---|
| Virus strain | VR-2332 | Amervac | Lelystad | Lena |
| Sequence homology to E38 (Identity, %) | 58.2 | 88 | 88.9 | 79.2 |

### Preparative example 2. Codon pair deoptimization

Using the E38 virus as a parent strain, an attenuated infectious clone was prepared by applying codon pair deoptimization in the genome synthesis step.

As a gene to be a subject of codon pair deoptimization, NSP1 and ORF-7 were selected, respectively, and it was prepared by silent mutation for some of the nucleotide sequence according to the codon pair deoptimization principle using commonly known SAVE (Synthetic Attenuated Virus Engineering) program or SAVE (Synthetic Attenuated Virus Engineering) program developed by the present inventors.

At first, a CPB (Codon Pair Bias) value, which is a bias that occurs when gene codons of the E38 (domestic isolated species) virus interact in pairs, was quantified using a computer algorithm. The CPB numerical value is known to fluctuate when some nucleotide sequences of the virus gene are substituted with other nucleotides, and be closely related to proliferation of the virus. When the CPB numerical value is reduced through nucleotide sequence substitution (deoptimization), the proliferation of the virus is reduced and attenuated.

### Comparative example 1. Preparation of codon deoptimization virus mutant for NSP1 region

### 1-1. Codon pair deoptimization

A gene region encoding NSP1 protein in PRRSV is known to have high genetic stability among the genome of the PRRSV. Therefore, codon pair deoptimization was applied for a nucleotide region showing a relatively high CPB value using SAVE program for the NSP1 gene region of the E38 (domestic isolated species) PRRSV.

The CPB, CpG and UpA numerical values before and after codon pair deoptimization for each strain designed by the method were shown in Table 2 below. The CpG and UpA numerical values are results necessarily occurring in the codon pair deoptimization process, and it is known that when the CpG or UpA numerical value increases in a gene, cell stress is induced, and therefore proliferation of a virus is reduced.

**[Table 2]**

| Parent strain | Codon deoptimization | CPB | CpG | UpA | nt |
|---|---|---|---|---|---|
| E38 | X (original) | -0.0511 | 0.7361 | 0.4878 | - |
| E38 | O (NSP1) | -0.2491 | 1.0859 | 0.8464 | 78 |

As can be confirmed in Table 2 above, it was suggested that as the result of applying codon deoptimization for the NSP1 gene, the CBP value was reduced in all the E38 and Lelystad virus strain, and the CpG and UpA numerical values were also increased, and therefore, the proliferation of the virus having the mutant genome as above was reduced, compared to a parent strain.

### 1-2. Confirmation of production of infectious strain of virus mutant

In order to produce the designed mutant strain, the genome (including codon deoptimized mutation) of the E38 or Lelystad strain was divided into 6 fragments to synthesize DNA, respectively. Each fragment was treated with restriction enzyme, and then linked with ligase to prepare one infectious clone.

The infectious clone was inserted into a high copy vector, and then transfected into BHK cell (Korean Cell Line Bank) using lipofectamine to try production of the infectious virus.

However, production of the E38 virus mutant in which codon deoptimization of the NSP1 gene was conducted was not achieved.

### Example 1. Preparation of codon deoptimized virus mutant for ORF7 region

### 1-1. Codon pair deoptimization of ORF7 region

ORF7 is a region present at the 3' end of the PRRSV genome, and refers to a region corresponding to the 398nt length at the 3' end region of the genome.

By the substantially same method as Comparative example 1-1, codon pair deoptimization for the ORF7 region against the E38 virus was performed using the SAVE program, and accordingly, a mutant in which a silent mutation was introduced at the ORF7 region was designed. In Table 3 below, the CPB, CpG, UpA and nt numerical values of the parent strain and mutant were shown. Description for each numerical value was as described in Comparative example 1-1 above.

**[Table 3]**

| Codon deoptimization | CPB | CpG | UpA | nt |
|---|---|---|---|---|
| X (parent strain) | -0.0371 | 0.5714 | 0.2610 | - |
| O (ORF7, 7) | -0.0963 | 0.7520 | 0.3589 | 7 |
| O (ORF7, 22) | -0.2251 | 1.0797 | 0.6185 | 22 |

In Table 3 above, the ORF sequences of the parent strain and each mutant were shown in SEQ ID NOs: 2 to 4. Specifically, SEQ ID NO: 2 is an ORF7 region nucleic acid sequence of the parent strain, and SEQ ID NO: 3 is an ORF7 region nucleic acid sequence of the strain comprising 7 mutations, and SEQ ID NO: 4 is an ORF7 region nucleic acid sequence of the strain comprising 22 mutations in Table 3 above.

The nucleic acid sequence of SEQ ID NO: 3 comprises silent mutations in which the 117th T is substituted with C, and the 180th T is substituted with C, and the 204th C is substituted with T, and the 213th T is substituted with G, and the 222th C is substituted with G, and the 264th A is substituted with C, and the 267th A is substituted with T, respectively, in the nucleic acid sequence of SEQ ID NO: 2.

The nucleic acid sequence of SEQ ID NO: 4 comprises silent mutations in which the 48th A is substituted with G, and the 108th C is substituted with G, and the 111th G is substituted with A, and the 123th C is substituted with T, and the 129th A is substituted with C, and the 153th T is substituted with C, and the 162th A is substituted with G, and the 171th C is substituted with G, and the 174th G is substituted with A, and the 177th T is substituted with C, and the 180th T is substituted with C, and the 186th T is substituted with C, and the 189th C is substituted with T, and the 204th C is substituted with T, and the 213th T is substituted with G, and the 222th C is substituted with G, and the 264th A is substituted with C, and the 267th A is substituted with T, and the 285th C is substituted with T, and the 288th T is substituted with C, and the 291th G is substituted with T, and the 297th C is substituted with T, respectively, in the nucleic acid sequence of SEQ ID NO: 2. In other words, the nucleic acid sequence of SEQ ID NO: 4 comprises all the mutations which SEQ ID NO: 3 has, and further comprises 15 mutations at the 48th, 108th, 111th, 123th, 129th, 153th, 162th, 171th, 174th, 186th, 189th, 285th, 288th, 291th and 297th positions additionally.

In case of the mutant using E38 as a parent strain, as the number of mutations introduced into ORF7 increased, the CPB numerical value decreased and the CpG and UpA numerical values increased.

### 1-2. Production of infectious strain of virus mutant

By the substantially same method as Comparative example 1-2, each virus mutant designed in Example 1-1 above (codon deoptimized virus mutant) was produced.

As a result, only in case of the mutant having ORF7 which consists of the nucleotide sequence of SEQ ID NO: 3, in which a silent mutation was introduced to 7 nucleotides, the production was successfully made, while the production of the mutant having ORF7 which consists of the nucleotide sequence of SEQ ID NO: 4, in which 22 silent mutations were introduced into the ORF7 of E38, was not achieved.

### Example 2. Virus production depending on the number and position of silent mutations in ORF7

### 2-1. Confirmation of virus production depending on the number of silent mutations

Based on the result of virus production in Example 1-2 above, in order to confirm whether the virus was produced dependently on the number of silent mutations for the E38 parent strain, by introducing silent mutations in which 14, 16, 18 and 20 nucleotides were further substituted through codon pair deoptimization, virus production was confirmed.

Specifically, the ORF7 nucleotide sequence of the mutant in which 14 nucleotides of ORF7 were substituted (E38_ORF7_CPD14_NUM_O) was shown in SEQ ID NO: 5, and the ORF7 nucleotide sequence of the mutant in which 16 nucleotides of ORF7 were substituted (E38_ORF7_CPD16_NUM_X) was shown in SEQ ID NO: 6, and the ORF7 nucleotide sequence of the mutant in which 18 nucleotides of ORF7 were substituted (E38_ORF7_CPD18_NUM_X) was shown in SEQ ID NO: 7, and the ORF7 nucleotide sequence of the mutant in which 20 nucleotides of ORF7 were substituted (E38_ORF7_CPD20_NUM_X) was shown in SEQ ID NO: 8.

More specifically, the nucleic acid sequence of SEQ ID NO: 5 comprises a silent mutation in which the 123th C was substituted with T, and the 129th A was substituted with C, and the 177th T was substituted with C, and the 180th T was substituted with C, and the 186th T was substituted with C, and the 189th C was substituted with T, and the 204th C was substituted with T, and the 213th T was substituted with G, and the 222th C was substituted with G, and the 264th A was substituted with C, and the 267th A was substituted with T, and the 288th T was substituted with C, and the 291th G was substituted with T, and the 297th C was substituted with T, in the ORF7 nucleic acid sequence of the parent strain (E38) represented by the nucleic acid sequence of SEQ ID NO: 2.

The nucleic acid sequence of SEQ ID NO: 6, comprises all the mutations which the SEQ ID NO: 5 has, and further comprises a mutation in which the 108th C was substituted with G and the 111th G was substitute with A additionally.

The nucleic acid sequence of SEQ ID NO: 7, comprises all the mutations which the SEQ ID NO: 5 has, and further comprises a mutation in which the 153th T was substituted with C, and the 162th A was substitute with G, and the 171th C was substituted with G, and the 174th G was substituted with A, additionally.

The nucleic acid sequence of SEQ ID NO: 8, comprises all the mutations which the nucleic acid sequences of SEQ ID NO: 6 and SEQ ID NO: 7 have. Specifically, it comprises all the mutations which the SEQ ID NO: 5 has, and further comprises a mutation in which the 108th C was substituted with G and the 111th G was substitute with A, and the 153th T was substituted with C, and the 162th A was substituted with G, and the 171th C was substituted with G, and the 174th G was substituted with A additionally.

The production of the virus was performed by the substantially same method as Comparative example 1-2, and only the E38_ORF7_cpd14_num_o mutant was successful in virus production, but the other three kinds of mutants (E38_ORF7_cpd16_num_x, E38_ORF7_cpd18_num_x and E38_ORF7_cpd20_num_x) failed in production.

Accordingly, it was confirmed that other factors except for the number of silent mutations were involved in virus production, when the silent mutation was 14 or more.

### 2-2. Virus production depending on silent mutation position

Based on the result of Example 2-1 above, in order to confirm whether the position of the silent mutation affects possibility of virus production, based on the mutation present in the common position of the virus having 22 mutations not produced in Example 1-2 and the E38_ORF7_cpd18_num_x used in Example 2-1 above, mutants in which 18 (E38_ORF7_cpd18_position_O), and 22 (E38_ORF7_cpd22_position_O) silent mutations were introduced in the ORF7 nucleotide sequence (SEQ ID NO: 2) of the parent strain were designed, respectively, and whether or not the virus was produced was confirmed.

The E38_ORF7_cpd18_position_O mutant has an ORF7 region consisting of the nucleic acid sequence of SEQ ID NO: 9, and specifically, it comprises a mutation in which C at the 123th position was substituted with T, and A at the 129th position was substituted with C, and T at the 177th position was substituted with C, and T at the 180th position was substituted with C, and T at the 186th position was substituted with C, and C at the 189th position was substituted with T, and C at the 204th position was substituted with T, and T at the 213th position was substituted with G, and C at the 222th position was substituted with G, and A at the 264th position was substituted with C, and A at the 267th position was substituted with T, and T at the 288th position was substituted with C, and G at the 291th position was substituted with T, and C at the 297th position was substituted with T, and T at the 309th position was substituted with C, and C at the 315th position was substituted with T, and T at the 327th position was substituted with C, and T at the 330th position was substituted with G, of the wild type ORF7 region consisting of the nucleic acid sequence of SEQ ID NO: 2.

The E38_ORF7_cpd22_position_O mutant has the orf7 sequence consisting of the nucleic acid sequence of SEQ ID NO: 10, and specifically, it comprises all the mutations which the E38_ORF7_cpd18_position_O strain has in the orF7 region of the wild-type virus consisting of the nucleic acid sequence of SEQ ID NO: 2, and further comprises a mutation in which A at the 336th position was substituted with G, and G at the 339th position was substituted with A, and T at the 348th position was substituted with A, and G at the 354th position was substituted with T additionally.

The production of each virus was performed by the substantially same method as Comparative example 1-2, and all the E38_ORF7_cpd18_position_O and E38_ORF7_cpd22_position_O strains succeeded in production, and the E38_ORF7_cpd22_position_O mutant was deposited to Korean Collection for Type Cultures (KCTC) on November 5^{th}, 2019, and received Accession number KCTC 14016BP.

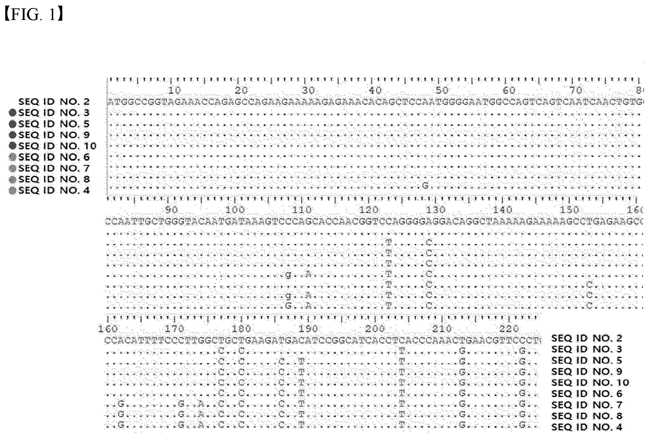

In FIG. 1, the sequence alignment result with respect to the mutation site of the ORF7 position of each virus designed in Examples 1-1 to 2-2 was shown. Differently from the E38_ORF7_CPD14_NUM_O mutant (ORF7 nucleic acid sequence: SEQ ID NO: 5) which succeeded in virus production, the production was not achieved in the mutant in which only the 108th and 111th nucleotides were further substituted in the ORF7 sequence of SEQ ID NO: 5 (E38_ORF7_cpd16_num_X, ORF7 nucleic acid sequence: SEQ ID NO: 6) and the mutant in which the 153th, 162th, 171th and 173th nucleotides were substituted in the ORF sequence of SEQ ID NO: 5 (E38_ORF7_CPD18_NUM_X). As the virus was not produced also in the E38_ORF7_cpd20_num_x comprising all the mutation positions of two mutants of the E38_ORF7_cpd16_num_X and E38_ORF7_cpd18_num_X and the virus mutant comprising the ORF7 region consisting of the nucleic acid sequence of SEQ ID NO: 4, it was confirmed that the 48th nucleotide, 108 to 111th nucleotides, or 153 to 173th nucleotides should be excluded from the subject of codon pair deoptimization in the nucleic acid sequence of SEQ ID NO: 5 which was the position in which each mutation was introduced.

After that, the strain in which the 309, 315, 327, 330th nucleotides after the 297th nucleotide were additionally substituted in the 14 nucleotides-substituted strain (E38_ORF7_cpd14_num_O) (18 nucleotides-substituted strain, e38_orf7_cpd18_position_o) was successfully produced, and the strain in which 4 nucleotides, the 336, 339, 348, 358th nucleotides were substituted in the ORF7 sequence of the 18 nucleotides-substituted strain (22 nucleotides-substituted strain, e38_orf7_cpd22_position_o) was successfully produced.

It was confirmed that codon pair deoptimization for obtaining an infectious clone of the attenuated European type (Type I) PRRSV through the data should apply a substitution region after the 177th nucleotide of the ORF7 region consisting of the nucleic acid sequence of SEQ ID NO: 2.

### Example 3. Confirmation of attenuation of virus mutant

Attenuation was confirmed *in vivo* for 4 kinds of viruses successful in production (E38_ORF7_cpd7_O, E38_ORF7_cpd14_num_o, E38_ORF7_cpd18_position_o, and E38_ORF7_cpd22_position_o). As a control group, E38 (original) which was a parent strain of the mutants was used.

After infecting the 4 kinds of mutants or parent strain into a porcine alveolar macrophage (PAM), respectively, the proliferation of each virus was confirmed by TCID₅₀ measurement.

More specifically, the PAM cell was aliquoted in a 6well plate comprising 2ml RPMI medium (comprising 10%(v/v) FBS, 1%(w/v) penicillin, streptomycin) at a ratio of 2 × 10⁶ cells/well per well. Then, the produced strain and parent strain were inoculated in wells different each other at a ratio of MOI 0.01, respectively, and in 1 hour after inoculation, all the supernatant was removed, and then the maintenance solution RPMI medium (same composition as above) 2ml was aliquoted. After that, every 1 day, 2 days, 3 days and 4 days, the supernatant of each well was collected and the TCID₅₀ (Tissue culture infective dose 50) was measured. For TCID₅₀, one that the day before the test, 2 × 10⁵ cell/well of MARC-145 cells were aliquoted with 100ul DMEM medium (10%(v/v) FBS, 1%(w/v) penicillin, streptomycin) into a 96 well plate and attached was used.

The collected supernatant was aliquoted in a row into the far left well of a 96 well plate by 200ul, and the DMEM medium (FBS, antibiotic-free) was aliquoted into the other well by 180ul. Then, from the left, 20ul each was collected with a multi-pipet, and the virus for inoculation was prepared by diluting it by decimal while aliquoting into the right well. It was diluted sequentially while changing tips, and the last 12^{th} well was maintained as a negative control group (supernatant-free DMEM medium).

The prepared virus diluted solution was inoculated in a MARC-145 cell-attached plate. Then, all the culture solution of the MARC-145 cell was removed, and PBS 200ul was aliquoted into each well, and the washing process was performed by repeating discarding 3 times. After that, the prepared virus diluted solution was inoculated by aliquoting it by 100ul per well, and in 2 hours after inoculation, all the inoculated solution was removed, and the new maintenance solution (DMEM medium, 10%(v/v) FBS, 1%(w/v) penicillin, streptomycin) was aliquoted by 100ul. For about 7 days after aliquoting, occurrence of clumping and apoptosis which is one kind of cytopathogenic effects (CPE) was confirmed in the cell.

As a final numerical value, based on the previous dilution factor of the well in which no CPE phenomenon was observed, the dilution factor at which 4 wells, half of the 8 virus inoculated solutions could show CPE was obtained.

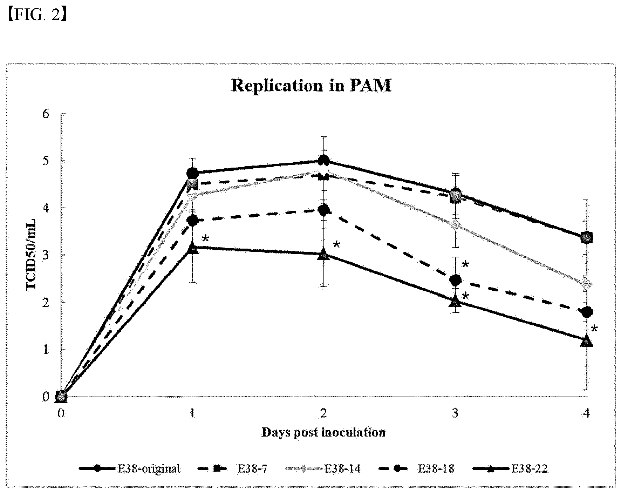

In FIG. 2, the TCID₅₀ analysis result graph was shown. In FIG. 2, the data marked as ^{∗} means that a statistically significant difference was shown, and each strain was expressed all in the form of 'number of E38-mutations'for convenience. All the 4 kinds of mutants showed lower proliferation compared to the parent strain (E38-original), and it was confirmed that the more codon pair deoptimization was done, the more attenuated, and in particular, the E38-18 and E38-22 strains were statistically significantly attenuated.

Specifically, the E38-22 strain showed a TCID₅₀/mL value at a level of about 65% compared to the parent strain at Day 1 after inoculation, and showed a TCID50/mL value at a level of about 35% at Day 4 after inoculation, and therefore, it was confirmed that it was significantly attenuated compared to the parent strain.

### Example 4. Analysis of proliferation (titer) in pigs of virus mutants

In order to analyze proliferation in pigs of each produced virus mutant, each virus was inoculated in pigs for an experiment, and then the RNA content of the virus in blood was measured using Real-time PCR. The information of the pig group used in each experiment was shown in Table 4 below.

**[Table 4]**

| Group | Inoculated strain | Number of pigs |
|---|---|---|
| T01 (control group) | E38 (Original) | 5 |
| T02 | E38_ORF7_cpd7_o | 5 |
| T03 | E38_ORF7_cpd14_num_o | 5 |
| T04 | E38_ORF7_cpd18_position_o | 5 |
| T05 | E38_ORF7_cpd22_position_o | 5 |

Pigs used in the experiment were SPF pigs, and 3-week-old piglets that were confirmed not to show any antigen-antibody reaction to PRRSV were used. Then, weaned 3-week-old piglets were inoculated with 5 TCID₅₀/2ml of each virus intranasally. On days 0 (on the day of inoculation), 7, 14, 21 and 28 from the inoculation day, 3ml of blood was collected from the jugular vein of the pig and serum was separated, and RNA of the virus comprised in the separated serum was extracted using Viral gene-spin viral DNA/RNA extraction kit of Intron company.

Specifically, at first, the collected serum 150ul was mixed with lysis buffer 250ul and it was vortexed for 15 seconds, and it was incubated at a room temperature for 10 minutes, and 350ul of binding buffer was mixed, and then it was vortexed softly. A total of 750ul mix was transferred to the column, and it was centrifuged at 13,000 rpm for 1 minute, and the filtrate was discarded, and washing buffer A 500ul was added to the column, and it was centrifuged at 13000rpm for 1 minute. The filtrate was discarded again, and washing buffer B 500ul was added to the column, and it was centrifuged at 13000rpm for 1 minute in the same manner. The last filtrate was discarded and it was centrifuged in the same manner, and all the alcohol components were removed. Elution buffer 30ul was added and it was incubated for 1 minute, and then centrifuged to finally extract RNA.

The extracted RNA 2ul was reverse-transcribed using TOPscript RT DRY MIX kit (Enzynomics) to synthesize cDNA. The reaction was terminated by mixing 2ul of the extracted RNA and DEPC DW 18ul into a tube of the kit already prepared in a dry state, and incubating at 50°C for 1 hour and then incubating at 95°C for 10 minutes.

Then, the amount of the synthesized cDNA genomic copy detected per serum 1ml using Realtime method was measured. The synthesized cDNA 2ul was added to the mix in which DEPC DW 7.5ul and the PRRSV specific primer set (SEQ ID NO: 11 to 12) 10pmol shown in Table 5 below were mixed in Sybr green mix (thermos fisher) 10ul and PCR reaction was performed. Specifically, DNA was amplified by conducting at 95°C for 30 seconds, annealing at 60°C, and elongation at 60°C for 1 minute, and additionally, a reaction for obtaining a dissociation curve was performed. A total of 35 cycle reactions were repeated, and a Ct value was calculated by sample according to the titer value and 33 or more were excluded. The Ct value was converted into a genomic copy by substituting into a standard curve and expressed.

**[Table 5]**

| PRRSV-specific primer | Nucleotide sequence (5'>3') | SEQ ID NO: |
|---|---|---|
| Forward primer (PRRSV forward) | TGGCCAGTCAGTCAATCAAC | 11 |
| Reverse primer (PRRSV reverse) | AATCGATTGCAAGCAGAGGGAA | 12 |

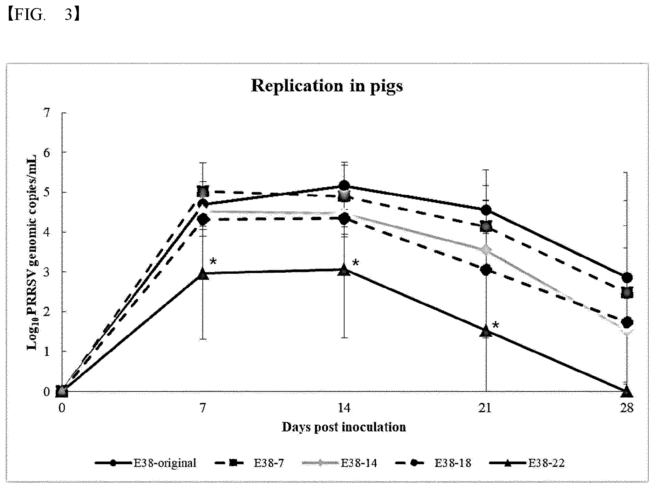

A graph of the blood virus titer over time after virus inoculation of each group was shown in FIG. 3. In FIG. 3, ^{∗} mark means a statistically significant result, and each virus was expressed in the form of 'number of E38-mutations' for convenience of description. As could be confirmed in FIG. 3, the blood virus titer of pigs measured at Day 7, Day 14 and Day 21 from the day of virus inoculation was measured statistically significantly lower than the conventional parent strain E38, when the E38_ORF7_cpd22_position_o was inoculated, and the lower titer at a level of 33% to 63% compared to the titer in the parent strain was maintained continuously after inoculated, and at Day 28, the titer of E38_ORF7_cpd22_position_o was measured as 0, and thereby, it was confirmed that the proliferation in pigs was very low.

Through this, it was confirmed that through codon pair deoptimization for the European PRRSV of the present invention, a safe strain which has a low maximum titer capable of proliferating in vivo and rapidly disappears could be made.

### [Accession Number]

Name of Depository Authority: Korean Culture Center of Microorganisms
Accession number: KCTC14016BP
Date of Deposit: 20191105

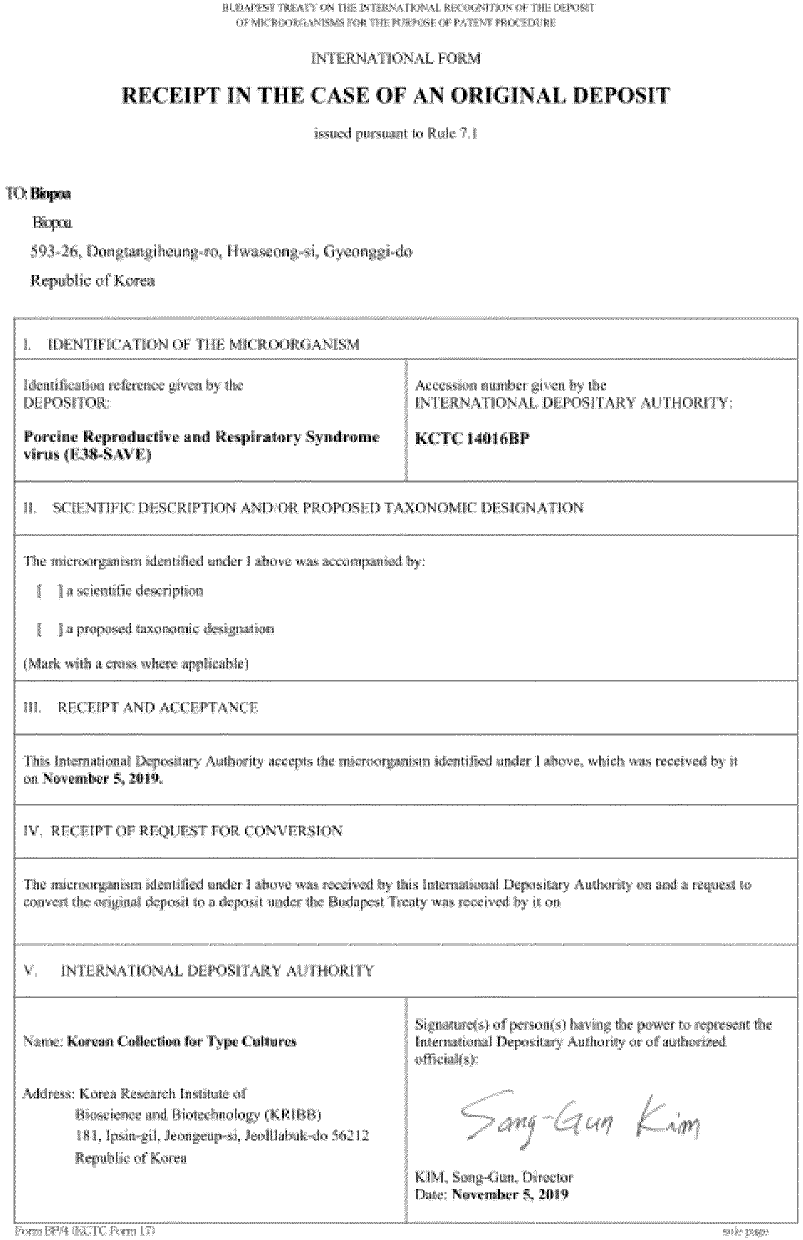
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Biopoa
Biopoa
593-26, Dongtangiheung-ro, Hwaseong-si, Gyeonggi-do
Republic of Korea

I. IDENTIFICATION OF THE MICROORGANISM
Identification reference given by the
DEPOSITOR: | Accession number given by the
INTERNATIONAL DEPOSITARY AUTHORITY:
Porcine Reproductive and Respiratory Syndrome
virus (E38-SAVE) | KCTC 14016BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on November 5, 2019.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):
KIM, Song-Gun, Director
Date: November 5, 2019
Form BP/4 (KCTC Form 17) sole page

## Claims

1. An attenuated porcine reproductive and respiratory syndrome (PRRS) virus mutant, having a low codon pair bias (CPB) compared to a porcine reproductive and respiratory syndrome (PRRS) virus,
wherein ORF7 consists of a nucleic acid sequence in which the nucleic acid sequence of SEQ ID NO: 2 is mutated by a silent mutation in the region after the 177th base.

2. The attenuated porcine reproductive and respiratory syndrome virus mutant according to claim 1, wherein the ORF7 consists of a nucleic acid in which the nucleic acid sequence of SEQ ID NO: 2 is mutated by one or more mutations selected form the following:
substitution of T at the 177th position with C, substitution of T at the 180th position with C, substitution of T at the 186th position with C, substitution of C at the 189th position with T, substitution of C at the 204th position with T, substitution of T at the 213th position with G, substitution of C at the 222th position with G, substitution of A at the 264th position with C, substitution of A at the 267th position with T, substitution of T at the 288th position with C, substitution of G at the 291th position with T, substitution of C at the 297th position with T, substitution of T at the 309th position with C, substitution of C at the 315th position with T, substitution of T at the 327th position with C, substitution of T at the 330th position with G, substitution of A at the 336th position with G, substitution of G at the 339th position with A, substitution of T at the 348th position with A, and substitution of G at the 354th position with G.

3. The attenuated porcine reproductive and respiratory syndrome virus mutant according to claim 2, wherein the ORF7 consists of a nucleic acid sequence in which the nucleic acid sequence of SEQ ID NO: 2 is mutated by the following mutation:
(1) a mutation including substitution of T at the 177th position with C, substitution of T at the 180th position with C, substitution of C at the 204th position with T, substitution of T at the 213th position with G, substitution of C at the 222th position with G, substitution of A at the 264th position with C, and substitution of A at the 267th position with T; or
(2) a combination of (i) the mutation of (1) above, and (ii) one or more mutations selected from the group consisting of substitution of T at the 186th position with C, substitution of C at the 189th position with T, substitution of T at the 288th position with C, substitution of G at the 291th position with T, substitution of C at the 297th position with T, substitution of T at the 309th position with C, substitution of C at the 315th position with T, substitution of T at the 327th position with C, substitution of T at the 330th position with G, substitution of A at the 336th position with G, substitution of G at the 339th position with A, substitution of T at the 348th position with A, and substitution of G at the 354th position with T.

4. The attenuated porcine reproductive and respiratory syndrome virus mutant according to claim 3, wherein the ORF7 consists of a nucleic acid sequence in which the nucleic acid sequence of SEQ ID NO: 2 is further mutated by substitution of C at the 123th position with T, substitution of A at the 129th position with C, or a combination thereof.

5. The attenuated porcine reproductive and respiratory syndrome virus mutant according to claim 1, wherein the porcine reproductive and respiratory syndrome virus is a European porcine reproductive and respiratory syndrome virus.

6. A pharmaceutical composition for preventing porcine reproductive and respiratory syndrome (PRRS), comprising one or more mutants selected from the group consisting of the attenuated porcine reproductive and respiratory syndrome virus mutant of any one claim of claim 1 to claim 5 and subcultured offspring thereof.

7. The composition according to claim 6, wherein the porcine reproductive and respiratory syndrome is a European porcine reproductive and respiratory syndrome.

8. The composition according to claim 6, wherein the composition is a vaccine composition.

9. The composition according to claim 8, wherein the vaccine is a live vaccine.

10. A kit for preventing a porcine reproductive and respiratory syndrome (PRRS), comprising one or more virus mutants selected from the group consisting of the attenuated porcine reproductive and respiratory syndrome virus mutant of any one claim of claim 1 to claim 5 and subcultured offspring thereof.

11. A preparation method of an attenuated porcine reproductive and respiratory syndrome virus, comprising introducing a silent mutation at a position after a base corresponding to the 177th from the 5' end based on the nucleic acid sequence of SEQ ID NO: 2 in ORF7 region in genome of a wild-type porcine reproductive and respiratory syndrome virus; and
producing a virus in which the mutation is introduced.

12. The preparation method of an attenuated porcine reproductive and respiratory syndrome virus according to claim 11, wherein the silent mutation comprises 7 or more mutations selected from the group consisting of substitution of C at the 123th position with T, substitution of A at the 129th position with C, substitution of T at the 177th position with C, substitution of T at the 180th position with C, substitution of T at the 186th position with C, substitution of C at the 189th position with T, substitution of C at the 204th position with T, substitution of T at the 213th position with G, substitution of C at the 222th position with G, substitution of A at the 264th position with C, substitution of A at the 267th position with T, substitution of T at the 288th position with C, substitution of G at the 291th position with T, substitution of C at the 297th position with T, substitution of T at the 309th position with C, substitution of C at the 315th position with T, substitution of T at the 327th position with C, substitution of T at the 330th position with G, substitution of A at the 336th position with G, substitution of G at the 339th position with A, substitution of T at the 348th position with A, and substitution of G at the 354th position with T, based on the nucleic acid sequence of SEQ ID NO: 2.

13. The preparation method of an attenuated porcine reproductive and respiratory syndrome virus according to claim 12, wherein the silent mutation is, based on the nucleic acid sequence of SEQ ID NO: 2,
(1) a mutation including substitution of T at the 177th position with C, substitution of T at the 180th position with C, substitution of C at the 204th position with T, substitution of T at the 213th position with G, substitution of C at the 222th position with G, substitution of A at the 264th position with C, and substitution of A at the 267th position with T; or
(2) a combination of (i) the mutation of (1) above, and (ii) one or more mutations selected from the group consisting of substitution of T at the 186th position with C, substitution of C at the 189th position with T, substitution of T at the 288th position with C, substitution of G at the 291th position with T, substitution of C at the 297th position with T, substitution of T at the 309th position with C, substitution of C at the 315th position with T, substitution of T at the 327th position with C, substitution of T at the 330th position with G, substitution of A at the 336th position with G, substitution of G at the 339th position with A, substitution of T at the 348th position with A, and substitution of G at the 354th position with T.

14. The preparation method of an attenuated porcine reproductive and respiratory syndrome virus according to claim 13, wherein the silent mutation further comprises substitution of C at the 123th position with T, substitution of A at the 129th position with C, or a combination thereof, based on the nucleic acid sequence of SEQ ID NO: 2.

15. The preparation method of an attenuated porcine reproductive and respiratory syndrome virus according to any one claim of claim 11 to claim 14, wherein the porcine reproductive and respiratory virus is a European porcine reproductive and respiratory syndrome virus.
